# EUROPEAN PATENT SPECIFICATION

(11) **EP 4 061 342 B1**
(45) Date of publication and mention of the grant of the patent: **11.11.2026**
(21) Application number: 20812408.1
(22) Date of filing: 18.11.2020
(51) Int. Cl.: A61K 31/05, A61P 25/08

(54) **CANNABIDIOL-C1 (CBD-C1) FOR USE IN THE TREATMENT OF EPILEPSY**
CANNABIDIOL-C1 (CBD-C1) ZUR VERWENDUNG BEI DER BEHANDLUNG VON EPILEPSIE
CANNABIDIOL-C1 (CBD-C1) POUR SON UTILISATION DANS LE TRAITEMENT DE L'ÉPILEPSIE

(30) Priority: 19.11.2019 GB 201916846
(43) Date of publication of application: 28.09.2022
(73) Proprietor: Jazz Pharmaceuticals Research UK Limited, Sittingbourne, Kent ME9 8AG (GB)
(72) Inventor: GUY, Geoffrey, Sittingbourne, Kent ME9 8AG (GB); KNAPPERTZ, Volker, Sittingbourne, Kent ME9 8AG (GB); WHALLEY, Benjamin, Sittingbourne, Kent ME9 8AG (GB); WOOLLEY-ROBERTS, Marie, Sittingbourne, Kent ME9 8AG (GB)
(74) Representative: HGF
(86) International application number: PCT/GB2020/052938
(87) International publication number: WO 2021/099777

(56) References cited:
- WO-A1-2019/207319
- WO-A1-2019/219773
- SEUNG-HWA BAEK ET AL: "Boron trifluoride etherate on alumina-a modified Lewis acid reagent. An improved synthesis of cannabidiol", TETRAHEDRON LETTERS, ELSEVIER, AMSTERDAM , NL, vol. 26, no. 8, 1 January 1985 (1985-01-01), pages 1083 - 1086, XP001095309, ISSN: 0040-4039, DOI: 10.1016/S0040-4039(00)98518-6
- ROSENBERG EVAN C ET AL: "Therapeutic effects of cannabinoids in animal models of seizures, epilepsy, epileptogenesis, and epilepsy-related neuroprotection", EPILEPSY AND BEHAVIOR, ACADEMIC PRESS, SAN DIEGO, CA, US, vol. 70, 9 February 2017 (2017-02-09), pages 319 - 327, XP085033471, ISSN: 1525-5050, DOI: 10.1016/J.YEBEH.2016.11.006

## Description

### FIELD OF THE INVENTION

The present invention relates to a cannabidiol (CBD) type cannabinoid compound for use in the treatment of epilepsy, wherein the CBD type cannabinoid compound is cannabidiol-C1 (CBD-C1), wherein the CBD-C1 is in the form of a plant extract comprising at least 80% (w/w) CBD-C1 or is in the form of a synthetic compound, and wherein the dose of CBD-C1 is greater than 100 mg/kg/day. The scope of protection is defined by the appended claims.

The CBD-type cannabinoid, cannabidiol-C1 (CBD-C1), is a naturally occurring cannabinoid that can be found in minor quantities in the cannabis plant. Furthermore, the cannabinoid can be produced by synthetic means.

Disclosed herein are data which demonstrate the efficacy of CBD-C1 in models of disease. In addition, a method for the production of CBD-C1 is described.

### BACKGROUND TO THE INVENTION

Cannabinoids are natural and synthetic compounds structurally or pharmacologically related to the constituents of the cannabis plant or to the endogenous agonists (endocannabinoids) of the cannabinoid receptors CB1 or CB2. The only way in nature in which these compounds are produced is by the cannabis plant. Cannabis is a genus of flowering plants in the family *Cannabaceae,* comprising the species *Cannabis sativa, Cannabis indica,* and *Cannabis ruderalis* (sometimes considered as part of *Cannabis sativa*)*.*

Cannabis plants comprise a highly complex mixture of compounds. At least 568 unique molecules have been identified. Among these compounds are cannabinoids, terpenoids, sugars, fatty acids, flavonoids, other hydrocarbons, nitrogenous compounds, and amino acids.

Cannabinoids exert their physiological effects through a variety of receptors including, but not limited to, adrenergic receptors, cannabinoid receptors (CB1 and CB2), GPR55, GPR3, or GPR5. The principle cannabinoids present in cannabis plants are cannabinoid acids Δ9-tetrahydrocannabinolic acid (Δ9-THCA) and cannabidiolic acid (CBDA) with small amounts of their respective neutral (decarboxylated) cannabinoids. In addition, cannabis may contain lower levels of other minor cannabinoids. "Chemical composition, pharmacological profiling, and complete physiological effects of these medicinal plants, and more importantly the extracts from cannabis, remain to be fully understood." Lewis, M. M. et al., ACS Omega, 2, 6091-6103 (2017).

Crude extracts from cannabis plants containing CBD have been used by patients suffering from diseases and disorders. However, such crude products are unsuitable for use in pharmaceutical formulations. Those seeking to prepare more consistent CBD preparations for use in treating diseases or disorders have made a concerted effort to either prepare CBD synthetically or attempt to remove all compounds other than CBD, particularly psychoactive compounds such as THC, from plant derived cannabinoids. See for example US 2014/0298511.

The present invention encompasses the surprising discovery that a minor cannabinoid related to CBD has therapeutic efficacy. This compound, cannabidiol-C1 (CBD-C1) can be extracted from the cannabis plant and purified or may be produced synthetically.

As stated, cannabinoids are a class of compounds which may be derived naturally from the cannabis plant or produced synthetically via chemical synthesis.

More than 100 different cannabinoids produced by cannabis have been identified. These cannabinoids can be split into different groups as follows: phytocannabinoids; endocannabinoids and synthetic cannabinoids (which may be novel cannabinoids or synthetically produced versions of phytocannabinoids or endocannabinoids).

Phytocannabinoids are cannabinoids that originate from nature and can be found in the cannabis plant. Phytocannabinoids can be isolated from plants to produce a highly purified extract. Phytocannabinoids may be obtained as either the neutral (decarboxylated form) or the carboxylic acid form depending on the method used to extract the cannabinoids from plant material. For example, it is known that heating the carboxylic acid form will cause most of the carboxylic acid form to decarboxylate into the neutral form. Phytocannabinoids can only be produced from plants, however versions of phytocannabinoids may be produced synthetically via chemical synthesis.

Endocannabinoids are endogenous lipid-based retrograde neurotransmitters that bind to cannabinoid receptors, and cannabinoid receptor proteins that are expressed throughout the mammalian central nervous system (including the brain) and peripheral nervous system. The endocannabinoid system is involved in regulating a variety of physiological and cognitive processes including fertility, pregnancy, during pre- and postnatal development, appetite, pain-sensation, mood, and memory, and in mediating the pharmacological effects of cannabis.

Synthetic cannabinoids are compounds that have a cannabinoid-like structure and are manufactured using chemical means rather than by the plant.

Certain cannabinoids are described in more detail below.

Cannabidiol (CBD) is a major cannabinoid constituent of Cannabis species, such as the hemp plant (*Cannabis sativa*)*.* Unlike other cannabinoids, such as THC, cannabidiol does not bind CB1 or CB2, or its binding to the receptors is negligible in terms of inducing a pharmacological effect. Thus, cannabidiol does not cause the central or peripheral nervous system effects mediated by the CB1 or CB2 receptors. CBD has little or no psychotropic (cannabimimetic) activity and its molecular structure and properties are substantially different from those of other cannabinoids.

Cannabidiol administration has been the subject of research in an attempt to provide an alternative treatment for various diseases and disorders which may respond to such treatment.

Tetrahydrocannabinol (THC) is the principal psychoactive constituent of cannabis. THC is a partial agonist at the CB1 and CB2 receptors. Synthetic THC or dronabinol is approved for the treatment of loss of appetite in AIDS patients and nausea and vomiting caused by cancer chemotherapy.

Of the over 100 natural cannabinoids identified in *Cannabis sativa,* seven have been classified as CBD-type compounds, these cannabinoids have the same absolute configuration as CBD. These are: CBD, Cannabidiolic acid (CBDA), Cannabidivarin (CBDV), Cannabidivarin acid (CBDVA), Cannabidiol-C1 (CBD-C1), Cannabidiol-C4 (CBD-C4) and Cannabidiol monomethyl ether (CBDM).

Cannabidiolic acid (CBDA) is the main form in which CBD exists in the cannabis plant. It is converted into CBD after decarboxylation.

Cannabidivarin (CBDV) is a homolog of CBD, with the side-chain shortened by two methylene bridges. CBDV is a non-psychoactive cannabinoid and has been shown to have anti-convulsant activity in a mouse model of epilepsy.

Cannabidiol-C1 (CBD-C1) also known as cannabidiorcol is a homolog of CBD, with the side-chain shortened by four methylene bridges. CBD-C1 occurs naturally in plants producing CBD but has not been shown to have any therapeutic effects.

Cannabidiol-C4 (CBD-C4) also known as nor-cannabidiol is a homolog of CBD, with the side-chain shortened by one methylene bridge. CBD-C4 occurs naturally in plants producing CBD and prior to the present invention has not been shown to have any therapeutic effects.

Cannabidiol (CBD) preparations comprising greater than or equal to 98% (w/w) CBD and less than or equal to 2% (w/w) other cannabinoids, wherein the less than or equal to 2% (w/w) other cannabinoids comprise the cannabinoids tetrahydrocannabinol (THC); cannabidiol-C1 (CBD-C1); cannabidivarin (CBDV); and cannabidiol-C4 (CBD-C4), have been disclosed for their use in the treatment of epilepsy (WO 2019/207319 A1). The present invention demonstrates data for the first time to indicate that the compound cannabidiol-C1 may have therapeutic benefit.

### BRIEF SUMMARY OF THE DISCLOSURE

In accordance with the present invention there is provided cannabidiol-C1 (CBD-C1) for use in the treatment of epilepsy, wherein the CBD-C1 is in the form of a plant extract comprising at least 80% (w/w) CBD-C1 or is in the form of a synthetic compound, and wherein the dose of CBD-C1 is greater than 100 mg/kg/day.

Preferably the CBD-C1 is in the form of a plant extract. More preferably the CBD-C1 is in the form of a highly purified extract of cannabis.

The highly purified extract comprises at least 80% (w/w) CBD-C1, more preferably the highly purified extract comprises at least 85% (w/w) CBD-C1, more preferably the highly purified extract comprises at least 90% (w/w), more preferably the highly purified extract comprises at least 95% (w/w) CBD-C1, more preferably still the highly purified extract comprises at least 98% (w/w) CBD-C1.

Alternatively, the CBD-C1 is present as a synthetic compound.

The dose of CBD-C1 is greater than 100 mg/kg/day. More preferably the dose of CBD-C1 is greater than 250 mg/kg/day. More preferably the dose of CBD-C1 is greater than 500 mg/kg/day. More preferably the dose of CBD-C1 is greater than 750 mg/kg/day. More preferably the dose of CBD-C1 is greater than 1000 mg/kg/day. More preferably the dose of CBD-C1 is greater than 1500 mg/kg/day.

Also in accordance with the present invention there is provided a composition comprising cannabidiol-C1 (CBD-C1), and one or more pharmaceutically acceptable excipients for use in the treatment of epilepsy, wherein the CBD-C1 is in the form of a plant extract comprising at least 80% (w/w) CBD-C1 or is in the form of a synthetic compound, and wherein the dose of CBD-C1 is greater than 100 mg/kg/day.

Preferably the epilepsy is treated in a mammal. More preferably the mammal is a human. Alternatively, the mammal is a dog.

Further disclosed is a method for the production of cannabidiol-C1.

### BRIEF DESCRIPTION OF THE DRAWINGS

Embodiments of the invention are further described hereinafter with reference to the accompanying drawings, in which:
Figure 1 shows the evaluation of CBD-C1 in the MEST test in the mouse as described in Example 2.
Figure 2 shows the effect of CBD-C1 on the electroshock-induced generalised seizure threshold (MEST) in the mouse as described in Example 3.

### CANNABINOIDS AND THEIR ABBREVIATIONS

The cannabinoids described in the present application are listed below along with their standard abbreviations.

| | | |
|---|---|---|
| CBD | Cannabidiol | 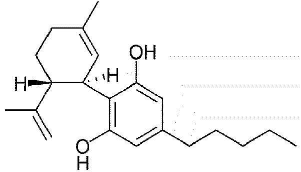 |
| CBD-C1 | Cannabidiol-C1 | 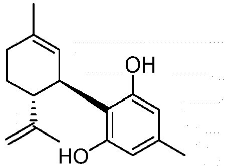 |

### DETAILED DESCRIPTION

### EXAMPLE 1: SYNTHETIC PRODUCTION METHOD FOR CANNABIDIOL-C1 (CBD-C1)

As previously described the compound CBD-C1 is produced as a minor cannabinoid in the cannabis plant. In a highly purified extract of cannabidiol the amount of CBD-C1 which remains in the extract is not more than 0.15% (w/w).

As such the synthetic pathway described below details a methodology that can be used in order to produce the cannabinoid CBD-C1 in larger quantities.

On the scheme R=CH₃

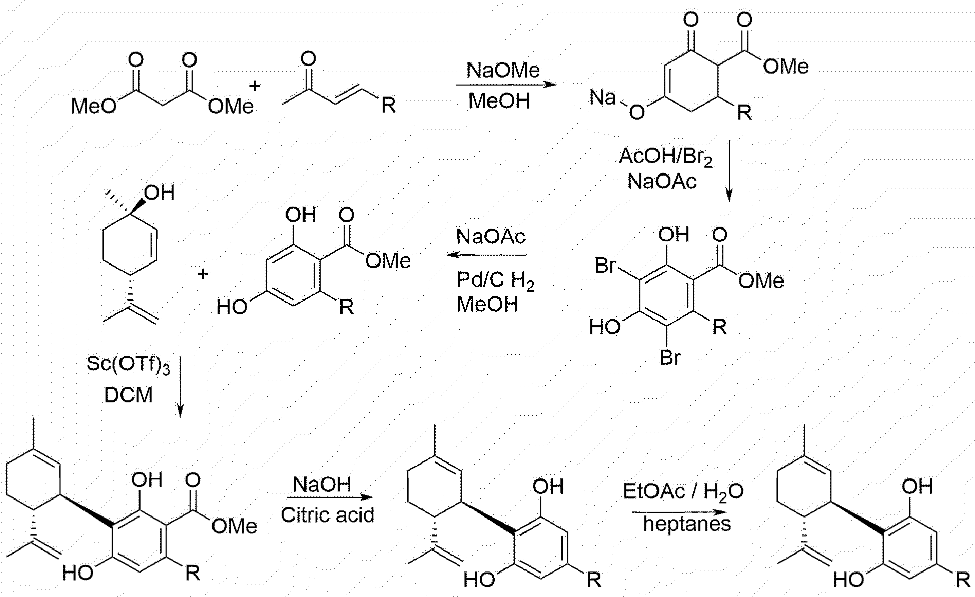

### EXAMPLE 2: EVALUATION OF CANNABIDIOL-C1 (CBD-C1) FOR ANTICONVULSANT ACTIVITY USING THE MAXIMAL ELECTROSHOCK SEIZURE THRESHOLD (MEST) TEST IN THE MOUSE

The efficacy of CBD-C1 was tested in a mouse model of seizure, the maximal electroshock seizure threshold (MEST) test.

The maximal electroshock seizure threshold (MEST) test is widely utilized preclinically to evaluate pro- and anti-convulsant properties of molecules (Loscher et al., 1991).

In the MEST test the ability of a drug to alter the seizure threshold current required to induce hind limb tonic extensor convulsions is measured according to an "up and down" method of shock titration (Kimball et al., 1957). An increase in seizure threshold is indicative of anti-convulsant effect. Antiepileptic drugs including the sodium channel blockers (e.g. lamotrigine) with clinically proven efficacy against generalised tonic-clonic seizures all exhibit anti-convulsant properties in this test in the mouse.

Conversely, a reduction in seizure threshold is indicative of a pro-convulsant effect as observed with known convulsant agents such as picrotoxin.

### Methods

Naïve mice were acclimatised to the procedure room in their home cages, with food and water available ad libitum.

Animals were dosed i.p. according to dose group.

The vehicle (10ml/kg i.p. 60 min pre-treatment time) was 1:1:18 vehicle 5% ethanol, 5% kolliphor EL, 90% saline.

The test compound, CBD-C1 was administered at doses of 20, 100 and 200mg/kg given at 10ml/kg i.p. 60min pre-treatment time.

The positive control diazepam was used at 2.5mg/kg (10ml/kg i.p. 30min pre-treatment time)

Mice were individually assessed for the production of a tonic hind limb extensor seizure using a Hugo Sachs Electronik stimulator, which delivered an adjustable constant current (1-300 mA) of 0.1 s duration via corneal electrodes.

The stimulus intensity was varied by an 'up and down' method of shock titration. Thus, the first mouse within a treatment group was given a shock at the expected or estimated seizure threshold (CC₅₀) current, that is, the current producing tonic hind limb extensor seizure in 50% of animals. For subsequent animals, the stimulus intensity was lowered or raised in 2mA intervals if the preceding mouse did or did not show tonic hind limb extension, respectively.

This procedure continued for all mice within the treatment group. Data generated from treatment group of n=12 was used to calculate the CC₅₀ ± s.e.m. values according to the method of Kimball et al. (1957).

Animal were culled immediately by concussion of the brain by striking the cranium, followed by dislocation of the neck.

Induction of seizure is measured as an all-or-nothing effect scored as either present (+) or absent (0) for each animal.

The data for each treatment group were recorded as the number of +'s and 0's at each current level employed and this information is then used to calculate the CC₅₀ value (current required for 50% of the animals to show seizure behaviour) ± standard error.

Data was analysed by comparing treated groups with the appropriate vehicle control using Fisher's Exact Probability tests.

### Results

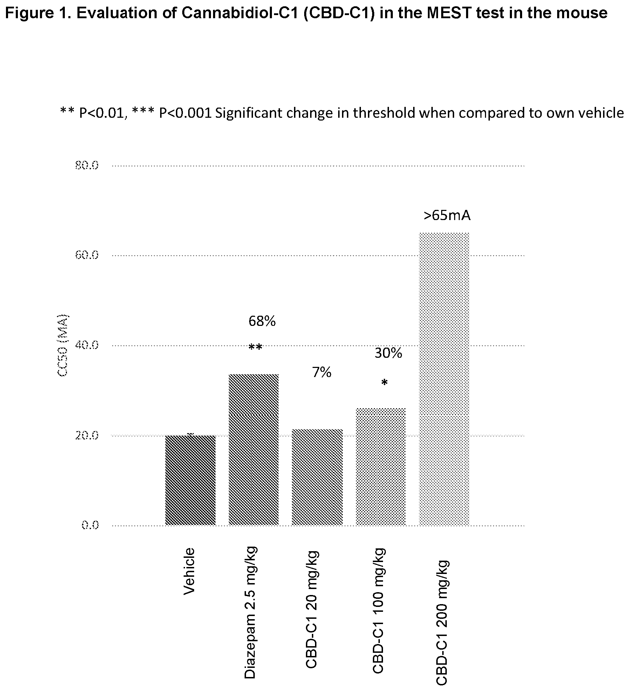

Figure 1 and Table 1 below demonstrates the data produced in this experiment.

In the vehicle group, the CC₅₀ value was calculated to be 20mA.

In the diazepam (2.5 mg/kg) treated group, administered i.p. 30 minutes before the test, the CC₅₀ value was 33.5mA. This result was statistically significant (p<0.01) compared to the vehicle control.

In the CBD-C1 treatment groups, administered i.p. 60 minutes before the test, the lower dose of 20 mg/kg CBD-C1 produced a statistically significant CC₅₀ value compared to vehicle.

In the mice treated with the higher doses of CBD-C1 there was a very large (>225%) difference from vehicle and as such the significance value could not be calculated. However, the effect seen should be considered to be of therapeutic benefit.

**Table 1: Evaluation of effect of CBD-C1 in the MEST test**

| **Treatment** | **Dose (mg/kg)** | **N** | **CC₅₀ +/- SEM** | **Significance** | **% change from vehicle** |
|---|---|---|---|---|---|
| Vehicle | - | 12 | 20.0 +/- 0.4 | - | - |
| Diazepam | 2.5 | 12 | 33.5 +/- 4.7 | P<0.01 | 68% |
| CBD-C1 | 20 | 12 | 21.4 +/- 0.3 | P<0.05 | 7% |
| CBD-C1 | 100 | 12 | 26.0 +/- 6.4 | Non sig | 30% |
| CBD-C1 | 200 | 12 | >65.0 | | >225% |

### Conclusions

These data demonstrate for the first time a therapeutic effect for the compound CBD-C1.

These data are significant as they provide heretofore unknown evidence that this cannabinoid which is found in minor quantities in extracts of cannabis plant may be of therapeutic value.

### EXAMPLE 3: EVALUATION OF CANNABIDIOL-C1 (CBD-C1) FOR ANTICONVULSANT ACTIVITY USING THE MAXIMAL ELECTROSHOCK SEIZURE THRESHOLD (MEST) TEST IN THE MOUSE

The efficacy of CBD-C1 was tested in a mouse model of generalised seizure, the maximal electroshock seizure threshold (MEST) test, as in Example 2.

### Methods

### Study Details:

Naïve mice were acclimatised to the procedure room in their home cages for up to 7 days, with food and water available ad libitum.

All animals were weighed at the beginning of the study and randomly assigned to treatment groups based on a mean distribution of body weight across groups. All animals were dosed at 10 mL/kg via intraperitoneal (i.p) injection, with either vehicle, CBD-C1 at 50, 100 or 150 mg/kg, diazepam at 2.5 mg/kg or sodium valproate at 250 mg/kg.

Animals were individually assessed for the production of a tonic hind limb extensor convulsion at 15 min post-dose for vehicle, at 15, 15 and 30 min post-dose for CBD-C1 at 50, 100 and 150 mg/kg respectively, and 30 min post-dose for diazepam and sodium valproate, from a single electroshock.

The first animal within a treatment group was given a shock at the expected or estimated CC₅₀ current. For subsequent animals, the current was lowered or raised depending on the convulsions outcome from the preceding animal.

Data generated from each treatment group were used to calculate the CC₅₀ ± SEM values for the treatment group.

### Test Compounds:

Vehicle: (5% ethanol, 5% solutol, 90% Saline) was prepared as follows: 2 mL of ethanol, 2 mL of solutol were warmed to 60°C, in 36 mL of saline (1:1:18).

Positive controls: diazepam was used at 2.5mg/kg and sodium valproate at 250 mg/kg.

The test compound, CBD-C1 was prepared according to the method described in Example 1. CBD-C1 was administered at 50, 100 and 150 mg/kg (i.p.) in a 1:1:18 ethanol:solutol:0.9% saline formulation.

### Sample Collection:

Each animal was humanely killed immediately after production of a convulsion by destruction of the brain from striking the cranium, followed by the confirmation of permanent cessation of the circulation from decapitation under The Humane Killing of Animals under Schedule 1 to the Animals (Scientific Procedures) Act 1986. Terminal blood and brain collection were performed following decapitation.

Blood was collected in Lithium-heparin tubes and centrifuged at 4°C for 10 minutes at 1500 x g. The resulting plasma was removed (>100 µL) and split into 2 aliquots of 0.5 mL Eppendorf tubes containing 100 µL of ascorbic acid (100 mg/mL) for stabilisation. Brains were removed, washed in saline and halved. Each half was placed into separate 2 mL screw cap cryovials, weighed and frozen on cardice.

### Statistical analysis

The data for each treatment group were recorded as the number of +'s and 0's at each current level employed and this information is then used to calculate the CC₅₀ value (current required for 50% of the animals to show seizure behaviour) ± standard error.

CBD-C1 effects were also calculated as percentage change in CC₅₀ from the vehicle control group.
Significant difference between drug-treated animals and controls were assessed according to Litchfield and Wilcoxon (1949).

### Results

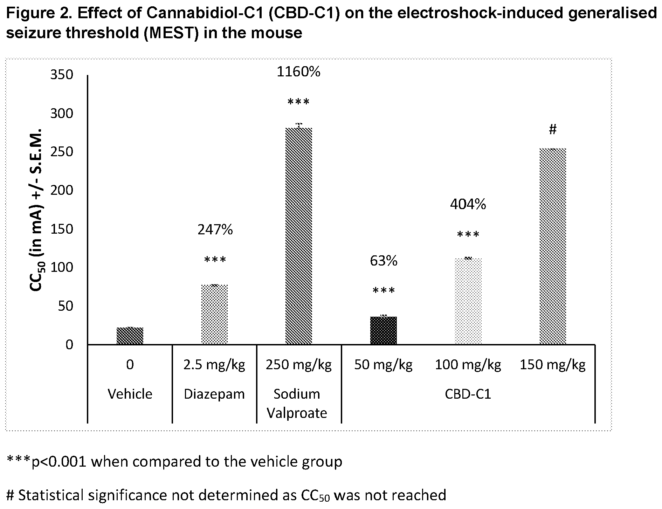

Table 2 below demonstrates the data produced in this experiment, and Figure 2 illustrates these results.

In the vehicle group, the CC₅₀ value was calculated to be 22.3mA.

In the positive control diazepam (2.5 mg/kg) treated group, administered i.p. 30 minutes before the test, the CC₅₀ value was 77.5mA. In the sodium valproate (250 mg/kg) treated group, administered i.p. 30 minutes before the test, the CC₅₀ value was 281.5mA. These results were statistically significant (p<0.001) compared to the vehicle control.

In the CBD-C1 treatment groups, administered i.p. 15 minutes before the test, the doses of 50 and 100 mg/kg CBD-C1 produced a statistically significant CC₅₀ value compared to vehicle. CBD-C1 tested at 150 mg/kg produced a CC₅₀ > 255; an exact value was not calculated as a "+" tonic hindlimb convulsion was not seen within the 12 animals tested. Although CC₅₀ was not determined and statistical significance was not achieved, 150 mg/kg showed a clear increase in seizure threshold in the MEST.

Such data are indicative that this compound will be of therapeutic benefit.

**Table 2: Evaluation of effect of CBD-C1 in the MEST test**

| **Treatment** | **Dose (mg/kg)** | **N** | **Pre-treatment time (mins)** | **CC₅₀ ± SEM** | **% change from vehicle** | **Significance** |
|---|---|---|---|---|---|---|
| Vehicle | - | 12 | 15 | 22.3± 0.4 | - | - |
| Diazepam | 2.5 | 12 | 30 | 77.5±0.4 | 247% | P<0.001 |
| Sodium Valproate | 250 | 12 | 30 | 281.5 ± 5.8 | 1160% | P<0.001 |
| CBD-C1 | 50 | 12 | 15 | 36.5±2.1 | 63% | P<0.001 |
| CBD-C1 | 100 | 12 | 15 | 112.5±0.7 | 404% | P<0.001 |
| CBD-C1 | 150 | 12 | 30 | >255 | >1042% | # |

| | | | | | | |
|---|---|---|---|---|---|---|
| # Statistical significance not determined as CC₅₀ was not reached. | | | | | | |

### Conclusions

CBD-C1 produced a dose-related increase in MEST, which provides evidence that this compound exhibits anticonvulsive properties. Significant effects were observed at 50 and 100 mg/kg, when compared to vehicle.

These data are significant as they provide heretofore unknown evidence that this cannabinoid may be of therapeutic value.

## Claims

1. Cannabidiol-C1 (CBD-C1) for use in the treatment of epilepsy, wherein the CBD-C1 is in the form of a plant extract comprising at least 80% (w/w) CBD-C1 or is in the form of a synthetic compound, and wherein the dose of CBD-C1 is greater than 100 mg/kg/day.

2. CBD-C1 for use according to claim 1, wherein the plant extract comprises at least 95% (w/w) CBD-C1.

3. A composition for use in the treatment of epilepsy comprising cannabidiol-C1 (CBD-C1) and one or more pharmaceutically acceptable excipients, wherein the CBD-C1 is in the form of a plant extract comprising at least 80% (w/w) CBD-C1 or is in the form of a synthetic compound, and wherein the dose of CBD-C1 is greater than 100 mg/kg/day.

4. CBD-C1 for use according to claim 1 or claim 2, wherein the epilepsy treated is in a mammal.

5. CBD-C1 for use according to claim 4, wherein the mammal is a human.

6. CBD-C1 for use according to claim 4, wherein the mammal is a dog.

## Patentansprüche

1. Cannabidiol-C1 (CBD-C1) zur Verwendung bei der Behandlung von Epilepsie, wobei das CBD-C1 in der Form eines Pflanzenextrakts vorliegt, der mindestens 80 Gew.-% CBD-C1 umfasst, oder in der Form einer synthetischen Verbindung vorliegt, und wobei die Dosis von CBD-C1 größer als 100 mg/kg/Tag ist.

2. CBD-C1 zur Verwendung nach Anspruch 1, wobei der Pflanzenextrakt mindestens 95 Gew.-% CBD-C1 umfasst.

3. Zusammensetzung zur Verwendung bei der Behandlung von Epilepsie, umfassend Cannabidiol-C1 (CBD-C1) und einen oder mehrere pharmazeutisch unbedenkliche Hilfsstoffe, wobei das CBD-C1 in der Form eines Pflanzenextrakts vorliegt, der mindestens 80 Gew.-% CBD-C1 umfasst, oder in der Form einer synthetischen Verbindung vorliegt, und wobei die Dosis von CBD-C1 größer als 100 mg/kg/Tag ist.

4. CBD-C1 zur Verwendung nach Anspruch 1 oder Anspruch 2, wobei die behandelte Epilepsie bei einem Säugetier vorliegt.

5. CBD-C1 zur Verwendung nach Anspruch 4, wobei das Säugetier ein Mensch ist.

6. CBD-C1 zur Verwendung nach Anspruch 4, wobei das Säugetier ein Hund ist.

## Revendications

1. Cannabidiol-C1 (CBD-C1) pour son utilisation dans le traitement de l'épilepsie, dans lequel le CBD-C1 se présente sous la forme d'un extrait de plante comprenant au moins 80 % (p/p) de CBD-C1 ou se présente sous la forme d'un composé synthétique, et dans lequel la dose de CBD-C1 est supérieure à 100 mg/kg/jour.

2. CBD-C1 pour son utilisation selon la revendication 1, dans lequel l'extrait de plante comprend au moins 95 % (p/p) de CBD-C1.

3. Composition pour son utilisation dans le traitement de l'épilepsie, comprenant du cannabidiol-C1 (CBD-C1) et un ou plusieurs excipients acceptables pharmaceutiquement, dans laquelle le CBD-C1 se présente sous la forme d'un extrait de plante comprenant au moins 80 % (p/p) de CBD-C1 ou se présente sous la forme d'un composé synthétique, et dans laquelle la dose de CBD-C1 est supérieure à 100 mg/kg/jour.

4. CBD-C1 pour son utilisation selon la revendication 1 ou la revendication 2, dans lequel l'épilepsie traitée concerne un mammifère.

5. CBD-C1 pour son utilisation selon la revendication 4, dans lequel le mammifère est un humain.

6. CBD-C1 pour son utilisation selon la revendication 4, dans lequel le mammifère est un chien.
